# EUROPEAN PATENT APPLICATION

(11) **EP 2 184 600 A2**
(43) Date of publication of application: **12.05.2010**
(21) Application number: 09175633.8
(22) Date of filing: 11.11.2009
(51) Int. Cl.: G01N 21/88

(54) **Surface quality determination of an object**

(30) Priority: 11.11.2008 SE 0850074
(71) Applicant: Stora Enso AB, 791 80 Falun (SE)
(72) Inventor: Wigge, Bo, 783 50, Gustafs (SE); Kolseth, Petter, 791 30, Falun (SE)

(57) **Abstract**

The present invention concerns a device and method for measuring the surface quality of an object. The device includes a portable object inspection entity (12) including a chamber (18) having a top surface (20), a bottom surface (22), and at least one side wall (24) joining the bottom surface with the top surface, a light detecting unit (34) directed towards an object inspection opening (26) provided in the bottom surface (22) of the chamber, thereby defining a line of sight (36) of the light detecting unit stretching through the chamber and being perpendicular to the object inspection opening, and a set of light sources (32) placed regularly around the line of sight within the chamber, where the light sources are directed towards the line of sight, in parallel with the object inspection opening.

## Description

### FIELD OF INVENTION

The present invention relates to device and method for measuring the surface quality of an object.

### BACKGROUND

It is in many cases of interest to inspect the surface quality of objects, for instance paper. This may be the case when for instance inspecting the paper quality of a batch of paper produced in a pulp and paper mill. However it may also be of interest to investigate such a quality at other premises, such as at the premises of a retailer. It would therefore be good if at least part of a device for measuring the surface quality could be portable and brought to such premises in order to enable the evaluation of the surface property of an object locally.

These types of devices are in many cases big and bulky, which make the inspection process burdensome.

It would therefore be of interest to provide the ability to inspect an object, like for instance a piece of paper or cardboard, with a device for measuring the surface quality of an object, where at least one entity of the device is portable.

There exist a few such surface inspection devices that are portable.

Some examples are given in US 5,377,000, US 5,724,139, US 7,227648 and WO 02/093109.

All of these devices use light sources that are in some way directed towards the object that is to be inspected.

When providing a device for measuring the surface quality of an object where at least one entity of the device is portable, it is of interest to make this portable entity as small as possible. This necessarily means that the light sources used will end up fairly close to the object being inspected.

However, when this is done with a light source that is directed towards a surface of the object, the gloss of the surface may become uneven. Dots with high gloss may occur and they may then be detected. This also means that the quality of the surface may not be correctly determined.

There is therefore a need for an improvement in the field of measuring the surface quality of an object.

### SUMMARY OF THE INVENTION

The present invention is therefore directed towards providing an improved device and method for measuring the surface quality of an object.

One object of the present invention is therefore to provide an improved device for measuring the surface quality of an object.

This object is according to a first aspect of the present invention solved through a device for measuring the surface quality of an object, for instance a paper, comprising:
a portable object inspection entity including a chamber having a top surface, a bottom surface, and at least one side wall joining the bottom surface with the top surface,
a light detecting unit directed towards an object inspection opening provided in the bottom surface of the chamber, thereby defining a line of sight of the light detecting unit stretching through the chamber and being perpendicular to the object inspection opening, and
a set of light sources placed regularly around said line of sight within said chamber,
wherein the light sources of said set are directed towards said line of sight, in parallel with said object inspection opening.

Another object of the present invention is to provide an improved method for measuring the surface quality of an object.

This object is according to a second aspect of the present invention solved through a method for measuring the surface quality of an object, comprising the steps of:
- emitting light directed towards a line of sight of a light detecting unit from a number of light sources regularly placed around and being directed towards said line of sight, and
- detecting light in said light detecting unit at one end of said line of sight, after reflection on the surface of an object to be inspected placed at an opposite end of said line of sight.

The present invention has a number of advantages. Through the claimed illumination according to the invention it is possible to provide the light sources closer to the object and thus the size of the portable object inspection entity can be reduced, which makes it easier to carry it around. This is furthermore done while at the same time guaranteeing satisfactory measurement results.

According to one variation of the present invention the processing of image data of the object involves performing a Fourier transformation on said image data in order to obtain light intensity values grouped according to spatial wavelength, calculating a median value for a number of light intensity values in a number of spatial wavelength intervals of a spatial wavelength range, where the range (R) includes at least one interval, and summing up the median values of each such interval of the range in order to obtain an indication of the quality of the object.

This has the further advantage of providing a more reliable processing of an image of the object. With regard to mottle determinations it is then possible to obtain a mottle value that is less influenced by the high peak intensities from periodic variations normal mottle determinations.

According to another variation of the present invention there is provided calibration data comprising printing instructions for printing a calibration area on an object, said printing instructions including colour or grey scale codes, and when being used by a printer device on an object, forms a number of sets of fields with differing reflectance, where each field in a set provides one level of reflectance, said printing instructions further providing the fields of the sets evenly distributed interspersed with each other and with areas lacking such fields in a calibration area (68) on the surface of the object being sized for covering the object inspection opening.

This calibration data has the advantage of enabling both calibration of grey scale to reflectance and to correct uneven illumination. It also provides information about spatial distortion and resolution of an image that could be used for further image corrections.

### BRIEF DESCRIPTION OF THE DRAWINGS

The present invention will in the following be described with reference being made to the accompanying drawings, where
fig. 1 schematically shows a device according to one embodiment of the present invention with two entities, a portable object inspecting entity and a processing entity in the form of a computer,
fig. 2 shows a sectional view through the middle of the portable object inspection entity,
fig. 3 schematically shows a number of light emitting units with a diffusing element and a polarizing element inside the portable object inspection entity of the invention,
fig. 4 shows a flow chart of a number of method steps taken in a method of measuring the surface quality of an object according to the invention and being performed in the portable object inspection entity,
fig. 5 schematically shows a simplified internal organization of units in the computer,
fig. 6 shows a flow chart of a number of method steps taken in a method of processing a digital image of an object according to the invention and being performed in the processing entity,
fig. 7 schematically shows a two-dimensional presentation of the result of a Fourier transformation being performed on a digital image captured in the portable object inspection entity, and
fig. 8 schematically shows a calibration area that has been printed on an object in order to calibrate the device according to the present invention.

### DETAILED DESCRIPTION OF THE INVENTION

In the following, a detailed description of preferred embodiments of a device and a method according to the present invention will be given.

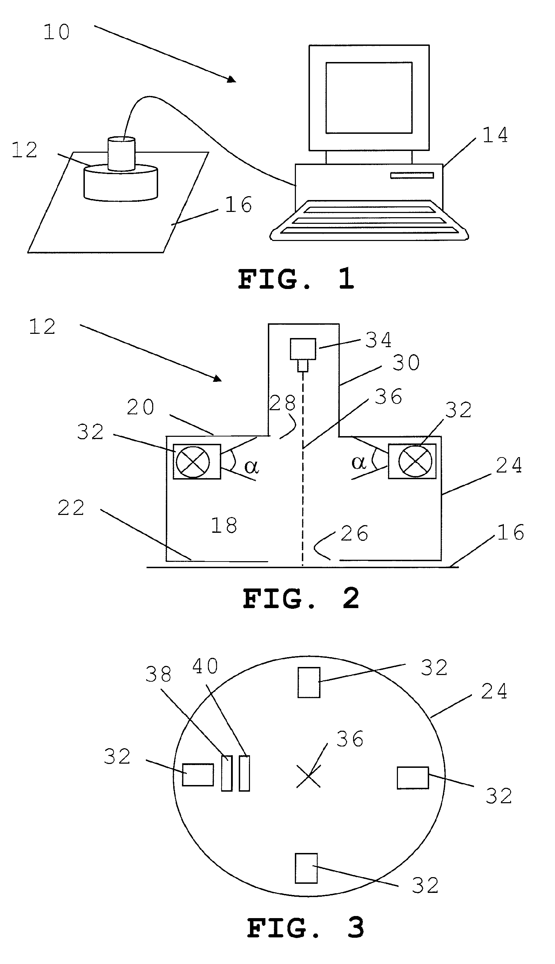

Figure 1 schematically shows a device 10 for measuring the surface quality of an object according to an embodiment of the present invention.

The device 10 includes a portable object inspection entity 12 which is connected to a processing entity 14, which processing entity is here provided in the form of a computer. The connection may be provided through a cable provided between for instance a USB port on the computer 14 and the portable object inspection entity 12.

For ergonomic purposes the portable object inspecting entity may be prepared for mounting with a weight-reducing hang-up or to be mounted in a stand for stationary work where the lifting of the portable object inspecting entity is controlled by a foot-pedal.

The portable object inspection entity 12 is adapted to be placed on an object 16, for instance a piece of paper or cardboard, the surface quality of which is to be inspected. In the portable object inspection entity 12 a light detecting unit, which is here a camera, captures a digital image of a part of the surface of the object 16. This image is then transmitted to the computer 14, in which an object surface quality determining unit or function processes the image in order to determine the surface quality of the object. Typical surface quality determinations are determinations regarding mottle or gloss.

The structure of the portable object inspection entity will now be described in more detail with reference being made to fig. 2, which shows a sectional view through the middle of the portable object inspection entity 12 and to fig. 3, which schematically shows a number of units inside the portable object inspection entity. The section in fig. 2 is here a section taken vertically through the middle of the portable inspection unit, while the view in fig. 3 is a view from above of some of the units in the interior of the portable entity.

The portable object inspection entity 12 according to an embodiment of the invention includes a chamber 18 having a top surface 20, a bottom surface 22 and one side wall 24 joining the bottom surface 22 with the top surface 20. The chamber 18 does here have a cylindrical shape and therefore the wall 24 is a circular wall. The chamber 18 does thus here have a circular cross-section. However, it should be realized that the present invention is not limited to this shape of the chamber, but that a chamber having for instance quadratic, rectangular, triangular or elliptical cross-section may be provided. There may thus be provided more walls.

The bottom surface 22, which is to be placed against an object 16 to be inspected, includes an object inspection opening 26. The top surface 20 of the chamber furthermore includes an opening 28. To this opening 28 there is joined a tube 30 aligned with and stretching from said top surface opening 28 in a direction away from the object inspection opening 26. The tube 30 is sealed in one end, the upper end that is located furthest from the bottom surface 22, and is open in the opposite end in order to be connected with the chamber 18. More importantly the tube 30 is also aligned with the object inspection opening 26. In the tube 30 there is provided a light detecting unit 34, which is here in the form of a digital camera. The camera may be a 1-chip colour camera or a monochrome or 3-chip camera. The camera 34 is furthermore directed towards the object inspection opening 26 in the bottom surface 22, and then preferably the centre of this object inspection opening. In this way there is defined a line of sight 36 of the camera 34 that stretches through the chamber 18 towards the object inspection opening 26. This line of sight 36 is thus perpendicular to the object inspection opening 26. The camera 34 is thus provided at one end of this line of sight 36, while the object inspection opening 26 is provided at the opposite end.

In the chamber there are furthermore provided a set of light sources 32, which are placed regularly around the line of sight 36 within the chamber 18 and here typically regularly spaced around the wall 24. They are furthermore each provided at the same distance from this line of sight. The number of light sources may vary. As an example in order to exemplify the present invention four are shown in fig. 3, while two are shown in fig. 2. It should be realized that many more may be provided such as for instance 24 or 30.

The light sources 32 are here light emitting diodes (LEDs) with a continuous spectrum. The power for the diodes may be supported through an external stabilized power-supply. This gives a very stable illumination. The diodes may be mounted on a flexible circuit board. This opens the possibility to change the design of the illumination for different purposes. For standard mottle measurements LEDs with visible light may be used.

The LEDs 32, which furthermore each has an aperture angle α of about 140 degrees, are furthermore directed towards the line of sight 36 of the camera. They are thus directed in parallel with the object inspection opening 26 and thereby with the surface of the object 16 to be inspected. Thereby these diodes 32 generally emit light in a direction perpendicular to the line of sight 36.

In front of each diode there may furthermore be provided a diffusing element 38 in order to diffuse the light emitted by a diode as well as a polarizing element 40. The diffusing element 38 is thus here provided between the LED and the polarizing element 40. In fig. 3 there is only shown one diffusing element 38 and one polarizing element 40 in front of one LED. It should however be realized that when these elements are used they are normally provided in front of each LED. These may each furthermore be provided as a strip of diffusing material and a strip of polarizing material provided around the wall 24 of the chamber 18. It should here be realized that it is also possible to provide colour filters in front of the LEDs. These colour filters would then be provided between the diffusing elements and the polarizing elements.

The functioning of the portable object inspection entity 12 will now be described with reference being made to fig. 2, 3 and 4, where the latter shows a flow chart of a number of method steps taken in a method of measuring the surface quality of an object according to the invention and being performed in the portable object inspection entity.

As the surface properties of an object, such as the reflective properties of a sheet of paper 16 are to be measured the portable object inspecting entity 12 is brought onto the object 16, such that the bottom side 22 is placed onto the object 16. The object should here be placed in close contact with the opening and should cover the whole of the opening so that not light will enter the chamber between the object and the opening. The camera 34 thus faces the object 16. Then the LEDs 32 emit light, step 42, which light is then emitted from the periphery of the chamber 18 towards the centre, i.e. towards the line of sight 36. When this is done the light is diffused by the diffusing element 38, step 44, and then plane polarized by the polarizing element 40, step 46. Thereafter the camera 34 detects light along the line of sight 36, step 48, and then light that has been reflected off the surface of the object 16 in the direction of the line of sight 36. This detected light is then captured as a digital image by the camera 34, which image is then sent from the camera 34 to the computer 14, step 50.

The way that the object is illuminated by the LEDs has a number of advantages. Since the LEDs, which are placed close to the object surface, are directed in parallel with this object surface and have a wide aperture angle, the gloss of the surface will become more even. In this way, dots with high gloss, which may otherwise appear in the image, are avoided. The diffusing element helps to provide an even intensity of the light that falls onto the object 16. The polarizing element 40, which thus polarizes the light in the plane attenuates gloss reflectance (e.g. for print mottle) and therefore also helps in obtaining a better result. Through this way of illuminating it is possible to provide the LEDs closer to the object and thus the size of the portable object inspection entity 12 can be reduced, which makes it easier to carry it around. This is furthermore done while at the same time guaranteeing satisfactory measurement results. Here an aperture angle of 140 degrees at the same time guarantees that enough light will reach the object surface.

There are a number of variations that can be made to the portable entity. LED for visible light could for instance be combined with LEDs in the UV-spectrum and thus make it possible to measure print/paper properties with or without UV or in only UV-light. The UV-light could be used for getting information on the distribution of optical brightener in a paper. The polarizer may be rotated by 90 degrees or replaced by a polarizing element with parallel polarization in order to enhance gloss. This may be done in order to measure print gloss variations instead of mottle. Of course also other filters can be included if needed. It is furthermore possible to provide the portable entity without diffusing element and/or without polarizing element. It is also possible to provide the portable entity with an image memory. In such a memory digital images may be temporarily stored. This allows the portable entity to be used separately from the computer in order to capture images of an object. When these images are then to be processed, the portable entity is brought to and connected to the computer and the captured images are loaded from the image memory to the computer for processing.

Now a description of the activities being performed by the computer will follow.

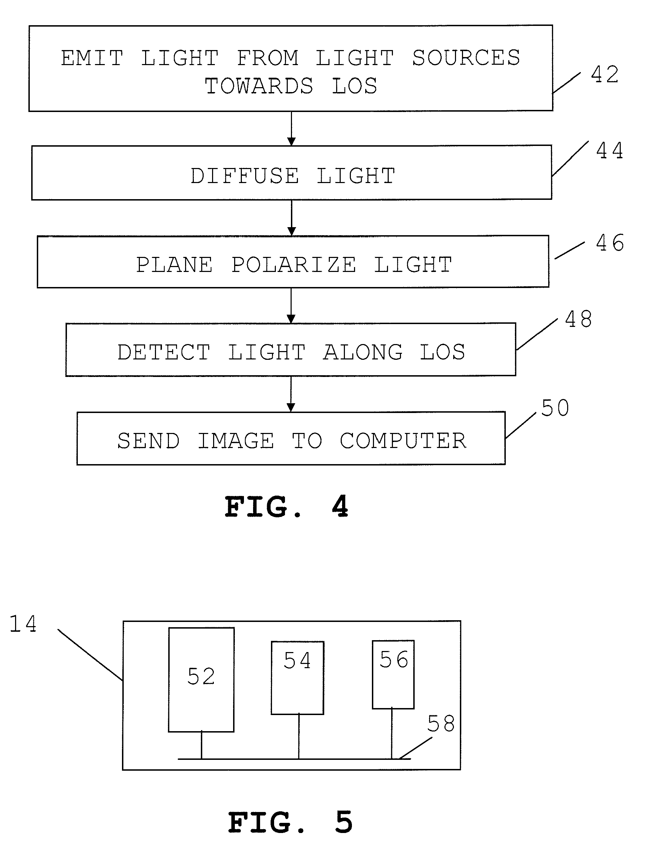

Fig. 5 schematically shows a simplified internal organization of units in the computer 14. The computer 14 includes a processor 52, a first program memory 54 and a second memory 56. All these units communicate with each other using a bus 58. In order to simplify the description of the present invention there is no communication interface to the portable object inspection entity shown or described. Since the use of for instance USB ports for communicating with other parts of a computer are well known within the art they need not be described any further. There is normally a communication interface connected to the bus 58 for providing such communication. Naturally there may be more units connected to the bus than what is shown in fig. 5.

The processing functionality used for investigating the surface quality of the object is according to an embodiment of the present invention provided in the form of a computer program including a number of computer program instructions. This computer program is according to this embodiment loaded in the program memory 54. The processor 52 here executes these program instructions. The combination of processor and program memory is therefore considered as an object surface quality determining unit.

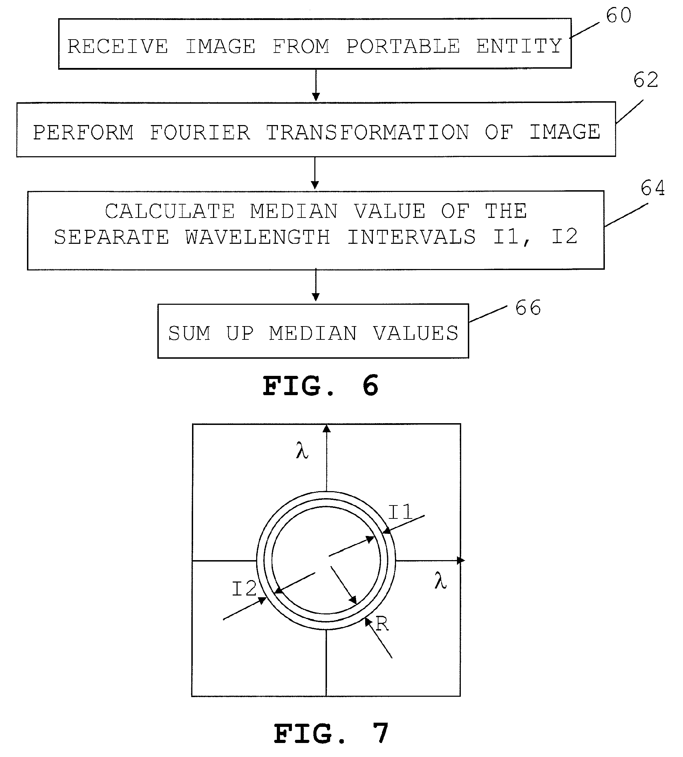

The general functioning of this surface quality determining unit will now be described with reference also being made to fig. 6, which shows a flow chart of a number of method steps taken in a method of processing a digital image of an object according to the invention and being performed by the surface quality determining unit, and to fig. 7, which schematically shows a two-dimensional graphical representation of the result of a Fourier transformation being performed on a digital image captured by the portable object inspection entity.

Image data corresponding to light detected by the camera in the form of the above-described digital image is thus transferred from the camera to the object surface quality determining unit 52, 54. The object surface quality determining unit 52, 54 thus receives the digital image from the camera in the portable entity, step 60. The image does, as is well known in the art include a number of pixels, each having a light intensity. The object surface quality determining unit 52, 54 processes the image in order to determine the surface quality of the object.

In the processing the object surface quality determining unit 52, 54 more particularly performs a Fourier transformation of the image, step 62. This is preferably performed using an FFT function. Through this transformation light intensity values grouped according to spatial wavelength are obtained. A two-dimensional representation of this is shown in fig. 7.

Light intensities are thus grouped according to spatial wavelength λ, where the variance of mottle in all directions is provided in an image. The spatial wavelengths λ are here provided along the X- and Y-axis. Thereafter the intensity values provided in a range R of spatial wavelengths are gathered in order to be analysed, for instance with regard to mottle. Now according to the present invention this range R is first divided into a number of spatial wavelength intervals, here exemplified by two intervals I1, I2. Thereafter a median value is calculated for the light intensity values of each interval I1, I2, step 64. Here there is thus calculated one median value for the first interval I1 and one median value for the second interval I2. Thereafter the median values of each interval in the range are summed up, step 66, in order to obtain an indication of the quality of the object, for instance concerning mottle.

Traditionally the range R has been handled through averaging the intensity values. However, if there are periodic patterns like moiré in the reflection off the object, these will have very high intensities at certain wavelengths. If mottle is calculated through averaging it will be highly influenced by this period pattern.

By instead calculating median values it is possible to obtain a mottle value that is less influenced by the high peak intensities from the periodic variations. The median will look at the middle value in a histogram and since the peak intensities caused by periodic variation will be present in a low amount compared to pixels from non-periodic variations, the variance due to random mottle will be even more spread out with lower intensities. The invention therefore also provides a more reliable processing of an image of the object.

It should here be realized that the above-described range may include only one interval, i.e. the range is the whole interval. Alternatively there may be several more intervals.

Since different scanners and cameras can give different results for measurements of e.g. mottle the object surface quality determining unit has a possibility to enter correction-factors to level the measurements according to accepted standards. This correction can be based either on a 2^{nd} degree fit or a lin-log curve.

The software used in the object surface quality determining unit may be modularly built. This makes it possible to add new calculations/measurement-routines. Also the image capturing functionality may be modular, which enables other cameras, scanners or file reading elements to be added and selected from the object surface quality determining unit. This means that several capturing elements can be used from the same computer.

The settings of the software can be saved both as "User-Settings" and as "Default". The default settings may be password-protected to avoid change by mistake.

In order to eliminate the risk of data loss, a backup may be created at each change of settings. These files can be loaded back to the software together with the settings. The data can then be printed out, saved to file or new measurements can be added.

The object surface quality determining unitcan save data as Excel-files or as text files.

In order to reduce the risk of entering incorrect sample names two features may be included: A list of sample names can be imported from a text file and a user may be allowed to select from this list. The software may also be prepared for a barcode reading.

Due to the modular design of the software it can easily be adopted to new cameras, new illuminations etc.

The surface quality determining unit was above being described as being provided as software loaded into a computer. This software may also be provided in the form of computer program code on a data carrier, such as a portable carrier, like a CD ROM disc or a memory stick, which performs the functionality of the object surface quality determining unit when said program code is loaded in the computer. Thus the processing entity may be a data carrier comprising the object surface quality determining unit in the form of computer program code.

The computer may furthermore be any type of computer like a PC, laptop or a palm top computer.

In order to calibrate the portable object inspection entity, there may according to the invention also provided a specially designed calibration area. This calibration area may be provided through calibration data, for instance in the form of a calibration data file, which may be stored in the second memory 56 shown in fig. 4. This file would then include printing instructions for printing calibration information on an object, like a sheet of paper. The printing instructions would then include colour or grey scale codes that when used by a printer device on an object, forms the calibration area.

This file may be invoked by the software with one command each time the surface quality determining unit is started or when a user makes major changes in settings. The calibration area may furthermore be mounted on the stand that also serves as protection. When the portable entity is not in use it may thus be placed on the stand and thereby both this entity and the calibration area are protected from mechanical damage and harmful light. During transport the stand may be locked to the portable entity.

The calibration area is provided for enabling both calibration of grey scale to reflectance and to correct uneven illumination. It also provides information about spatial distortion and resolution of the image that could be used for further image corrections.

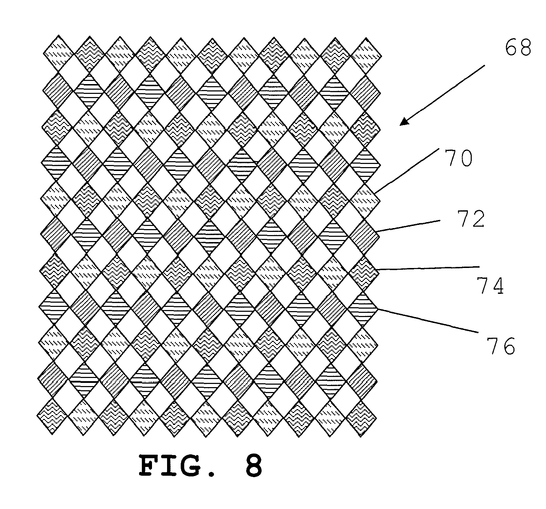

The calibration area 68 is schematically shown in fig. 8. It includes a number of sets of fields with differing reflectance, where each field is here diamond shaped, and each set has a different reflectance and thus provides one level of reflectance. There is thus a first set with fields 70 having a first reflectance, here caused by a first grey scale, a second set with fields 72 having a second reflectance here caused by a second grey scale, a third set with fields 74 having a third reflectance here caused by a third grey scale and a fourth set with fields 76 having a fourth reflectance here caused by a fourth grey scale.

The fields are furthermore evenly distributed interspersed with each other and with areas lacking such fields in the calibration area 68 on the surface of the object. The area 68 is here sized for covering the object inspection opening. In fig. 8 there is in more detail, as seen from top to bottom of the calibration area 68, a first row of fields including fields 70 of the first set with the first reflectance alternating with fields 74 of the third set with the third reflectance. The first row, which here includes eleven fields, furthermore starts and ends with a field 70 of the first set. Then a second row of fields including the fields 72 of the second set with the second reflectance alternating with fields 76 of the fourth set with the fourth reflectance follows. The row, which here also includes eleven fields, starts and ends with a field 72 of the second set. Thereafter follows a third row including the same alternating fields as the first row, but with the opposite order of the fields as compared with the first row. After the third row follows a fourth row including the same alternating fields as the second row, but with the opposite order of the fields as compared with the second row. After the fourth row follows a fifth row that is similar to the first row, etc. In this way the rows are varied until eleven rows are provided.

The area 68 thus provides an 11x11 lattice of fields, with the fields of the sets evenly distributed interspersed with each other, so that no fields of the same set are provided adjacent each other. As the fields are diamond-shaped there are furthermore diamond-shaped areas of non-printed material between the fields of the different rows. Every row thus includes fields from two sets. Every column of the lattice furthermore includes fields from all four sets in consecutive order after each other. There is however a shift of two fields in the fields of the same set between neighbouring columns. The fields 70 of the first set may here be the darkest fields, i.e. the fields with the lowest reflectance; the fields 72 in the second set may be the second darkest fields, i.e. the fields with the second lowest reflectance; the fields 74 in the third set may be the second brightest fields, i.e. the fields with the second highest reflectance and the fields 76 in the fourth set may be the brightest fields, i.e. the fields with the highest reflectance. They could of course also be provided the opposite way around so that the fields in the first set are the brightest and the fields in the fourth set the darkest. In this way the fields of the sets are evenly distributed interspersed with each other and with areas lacking such fields.

The area was above built up of diamond-shaped areas. However, it should be realized that other shapes may be provided, for instance circular, elliptical, quadratic or rectangular. It should here also be realized that more or fewer sets may be provided, for instance five. The lattice may furthermore include more or fewer rows and/or columns. The same levels of grey scale may also be present in a strip with larger fields, for instance rectangular or quadratic fields. These may be provided beside said lattice. The areas representing the object background may be slightly smaller than the printed fields and may not allow measurement of density. Since the demand of a paper may be that it must be very even in reflectance it is assumed that this measurement can be performed elsewhere on the sheet.

The calibration information provides both reflectance and evenness information. The following properties will furthermore be possible to estimate/perform:
- Reflectance
- Evenness of illumination/optics
- Resolution of pixels
- Geometrical distortion
- Optical resolution/Sharpness information.

### Reflectance calibration

The lattice holds four printed levels of reflectance. Together with the paper background it gives 5 levels to use for reflectance calibration. The darkest fields have been selected to ensure that reflectance will be above the black level of the camera for most normal settings.

A calibration routine in the surface quality determining unit will assume that the camera has a linear relationship to reflectance. Any deviations from this should be corrected for by for instance entering a correct Gamma Value. It should be realized that some cameras may have a linear relationship without such Gamma-correction.

### Evenness

By measuring the grey level/reflectance of each of the none-printed areas of the sheet a correction image can be created.

### Geometrical distortion

The non-printed areas are positioned in a regular spaced lattice. By fitting a lattice to the centre of each area and by comparing centre points to points of lattice an estimation of geometrical distortion can be made. This feature can be used to correct for warp in images.

### Pixel resolution

Since the distance in-between centre points of non-printed areas are known, the parameters from the lattice estimated for geometrical distortion can be used to estimate pixel resolution in X and Y direction, i.e. in the directions that rows and columns are provided in the lattice.

### Optical resolution/ Sharpness

By using the borders between bright and dark areas an estimation of the optical resolution can be obtained. For each border the slope of the edge can be calculated and by knowing the difference between border in grey levels or reflectance a measure can be obtained that gives the optical sharpness in µm. A perfect border is stated to have 0 µm spread.

### Import of calibration data into the computer

The calibration data can be added into the computer in 2 ways:
1. Values can be copied from Excel or a Text editor like notepad. In a text editor the columns should be separated by *Tab* and the rows separated by *CrLF.*
2. Values can be imported as a calibration file for instance named HMRyymmdd.txt. The file may be built like an ini-file according to description and example below:

### Example:

```
   ;Comments are marked with semicolon
   ;HandyMeasure Calibration Sheet 20031108 No. 0000001
   ; Measurements performed by BoW
   ;Darkest CalibrationArea is always named 0
   ;Indexes shall be in ascending order without
   ; leaving any number out.
   ;X is reflectance with X-filter expressed as %.
   ;
   [ID]
   IDNo=000001
   [Reflectance]
   X0=10,5
   Y0=10,6
   Z0=9,8
 
   X1=20,5
   Y1=20,6
   Z1=19,8
 
   X2=40,5
   Y2=40,6
   Z2=39,8
 
  X3=50,5
   Y3=50,6
   Z3=49,8
 
  X4=70,5
  Y4=70,6
   Z4=69,8
 
  X5=80,5
   Y5=80,6
   Z5=79,8
 
   [SpatialDivision]
   XDir=5 'Represents division in mm between centre of
   areas
   YDir=5
   [Evenness]; These are not used at present
   [Sharpness]
```

The file may also with advantage be provided on a data carrier, for instance together with the program code that is used for realizing the surface quality determining unit.

The present invention thus has a number of advantages. By providing a portable object inspection entity, where an object is illuminated by LEDs have a number of advantages. Since the LEDs directed in parallel with the object surface, the gloss of the surface will become more even. Through this way of illuminating it is possible to provide the LEDs closer to the object and thus the size of the portable object inspection entity can be reduced, which makes it easier to carry it around. This is furthermore done while at the same time guaranteeing satisfactory measurement results. Through calculating median values in the processing of a digital image in the object surface quality determining unit it is possible to obtain a mottle value that is less influenced by the high peak intensities from periodic variations. In this way a more reliable processing of an image of the object is provided. The calibration area has the advantage of enabling both calibration of grey scale to reflectance and to correct uneven illumination. It also provides information about spatial distortion and resolution of an image that could be used for further image corrections.

The present invention may be varied in a number of further ways apart from those already described. The various parts of the described invention may be provided separately. It is for instance possible to only provide the portable entity. It may then be combined with a known surface quality determining unit using averaging. The surface quality determining unit of the invention may likewise be used together with known object inspection entities, portable or fixed. The calibration data is likewise possible to use with such known surface quality determining unit and such known object inspection entity. The connection between the two entities need of course not be by cable. It is possible to use other means of communication, such as for instance Bluetooth.

## Claims

1. Device (10) for measuring the surface quality of an object (16), for instance a paper, comprising:
a portable object inspection entity (12) including a chamber (18) having a top surface (20), a bottom surface (22), and at least one side wall (24) joining the bottom surface with the top surface,
a light detecting unit (34) directed towards an object inspection opening (26) provided in the bottom surface (22) of the chamber, thereby defining a line of sight (36) of the light detecting unit stretching through the chamber and being perpendicular to the object inspection opening, and
a set of light sources (32) placed regularly around said line of sight within said chamber,
the light sources of said set are directed towards said line of sight, in parallel with said object inspection opening wherein the portable object inspection entity is adapted to be placed with the bottom side onto the object.

2. Device (10) according to claim 1, wherein the light sources (32) have an aperture angle (α) of 140 degrees.

3. Device (10) according to claim 1 or 2, further comprising at least one light diffusing element (38) between said set of light sources (32) and said line of sight (36).

4. Device (10) according to any previous claim, further comprising at least one polarizing element (40) between said set of light sources (32) and said line of sight (36).

5. Device (10) according to any previous claim, wherein the top surface (20) of the chamber (18) includes an opening (28) and further comprising a tube (30) aligned with and stretching from said top surface opening (28) in a direction away from said object inspection opening (26), where said light detecting unit (34) is provided in said tube.

6. Device (10) according to any previous claim, further comprising a processing entity (14) including an object surface quality determining unit (42, 44) arranged to receive image data corresponding to detected from said light detecting unit (34) of the portable object inspecting entity (12) and to process said image data in order to determine the surface quality of the object.

7. Device (10) according to claim 6, wherein the object surface quality determining unit, when being arranged to process the image data is arranged to perform Fourier transformation on said image data in order to obtain light intensity values grouped according to spatial wavelength,
calculate a median value for a number of light intensity values in a number of spatial wavelength intervals (I1, I2) of a spatial wavelength range (R), where said range (R) includes at least one interval (I1, I2), and sum up the median values of each such interval of the range in order to obtain an indication of the quality of the object.

8. Device (10) according to claim 6 or 7, wherein the processing entity is a data carrier and the object surface quality determining unit is provided in the form of computer program code on said data carrier performing the functionality of the object surface quality determining unit when said program code is loaded in a computer.

9. Device (10) according to claim 6 or 7, wherein the processing entity is a computer (14) and the object surface quality determining unit is provided as a processor (42) with program code in an associated program memory (44) performing the functionality of the object surface quality determining unit.

10. Device (10) according to any of claims 6 - 8, wherein the processing entity includes calibration data comprising printing instructions for printing a calibration area (68) on an object, said printing instructions including colour or grey scale codes, and when being used by a printer device on an object, forms a number of sets of fields with differing reflectance, where each field (70, 72, 74, 76) in a set provides one level of reflectance, said printing instructions further providing the fields of the sets evenly distributed interspersed with each other and with areas lacking such fields in a calibration area (68) on the surface of the object being sized for covering the object inspection opening.

11. Method for measuring the surface quality of an object by the use of the device described in any of claims 1-10, comprising the steps of:
- placing a portable object inspection entity with the bottom side onto the object,
- emitting (42) light directed towards a line of sight (36) of a light detecting unit (34) from a number of light sources (32) regularly placed around and being directed towards said line of sight, and
- detecting (48) light in said light detecting unit at one end of said line of sight, after reflection on the surface of an object (16) to be inspected placed at an opposite end of said line of sight.

12. Method according to claim 11, further comprising the step of diffusing (44) the light emitted from the light sources (32).

13. Method according to claim 11 or 12, further comprising the step of polarizing (46) the light emitted from the light sources (32).

14. Method according to any of claims 11 - 13, further comprising the steps of receiving (60) image data corresponding to said detected light in an object surface quality determining unit (52, 54) and processing (62, 64, 66) said image data in order to determine the surface quality of the object.

15. Method according to claim 14, wherein the processing of image data includes the further steps of: performing (62) Fourier transformation on said image data in order to obtain light intensity values grouped according to spatial wavelength (λ), calculating (64) a median value for a number of light intensity values in a number of spatial wavelength intervals (I1, I2) of a spatial wavelength range (R), where said range includes at least one interval, and summing up (66) the median values of each such interval (I1, I2) of the range (R) in order to obtain an indication of the quality of the object.
